# EUROPEAN PATENT APPLICATION

(11) **EP 3 692 922 A1**
(43) Date of publication of application: **12.08.2020**
(21) Application number: 19156524.1
(22) Date of filing: 11.02.2019
(51) Int. Cl.: A61B 7/00

(54) **A SYSTEM AND METHOD FOR IDENTIFYING A LOCATION OF THE HEART RESPONSIBLE FOR A PARTICULAR SOUND**

(71) Applicant: Koninklijke Philips N.V., 5656 AG Eindhoven (NL)
(72) Inventor: BERA, Deep, 5656 AE Eindhoven (NL); HIWALE, Sujitkumar Sureshrao, 5656 AE Eindhoven (NL); DE WILD, Nico Maris Adriaan, 5656 AE Eindhoven (NL); CHAKRABARTI, Biswaroop, 5656 AE Eindhoven (NL); GIJSBERS, Gerardus Henricus Maria, 5656 AE Eindhoven (NL); JOHNSON, Mark Thomas, 5656 AE Eindhoven (NL); RAMACHANDRAN, Ganesan, 5656 AE Eindhoven (NL)
(74) Representative: de Haan, Poul Erik

(57) **Abstract**

A system and method is for identifying a location of the heart responsible for a particular sound such as a heart murmur. Heart sounds are analyzed separately for heart cycles which take place during inspiration and during expiration. Based on the separate analysis, it is possible to determine if a particular heart sound is from the left side or the right side of the heart.

## Description

### FIELD OF THE INVENTION

This invention relates to the analysis of heart sounds, in particular to identify the location, such as the side, of the heart responsible for particular sounds.

### BACKGROUND OF THE INVENTION

Listening to the sound of the heart, auscultation, is a standard practice for diagnosing heart conditions. However, it requires significant training and expertise on the part of the medical practitioner. For example, different conditions affect different sides of the heart, and it is difficult to discriminate between sounds from the different sides of the heart. Different conditions also give rise to sounds at different times within the heart cycle, and this may again be difficult to interpret.

Several recent studies document the decline in physical examination skills among physicians. Reasons postulated for this shift are improvements in technology, time constraints, and uncertainty that stems from a lack of confidence in physical exam skills.

Standard equipment such as a stethoscope and tuning fork and methods for physical examination have been applied since the early 19th century. This equipment requires skill and practice to be used for objective assessment.

A cardiovascular examination is essentially an examination of the patient's heart; however, it is a complex examination which also includes examination of other parts of the body including the hands, face and neck. The cardiovascular examination aims to pick up on any cardiovascular pathology that may be causing a patient's symptoms, such as chest pain, breathlessness, or heart failure.

Key observations during the examination of the heart are (i) the heart rate, (ii) the size of the heart - measured by percussion and feeling the ictus of the heart, to determine the size of the heart as an indication for enlargement of the left ventricle, and (iii) valve function & blood flow - observed via auscultation of the heart at four standard positions, related to the different heart valves; mitral valve, aortic valve, tricuspid valve, pulmonary valve.

Heart sounds and murmurs give indications for valve defects, volume overload, pressure overload and hypertrophy.

As more emphasis is placed on technology, medical practitioners become less experienced and it becomes difficult to assess the source of a heart sound/murmur while auscultating the heart, depending on the frequency and amplitude of the heart sound. Doctors may not be able to distinguish between the systole (systolic phase) and diastole (diastolic phase. Auscultating sounds and/or left and right heart sounds are 0.01-0.02 seconds or less apart; so this becomes even more problematic when a person has high heart rate. Certain cardiac pathologies such as pleural effusion reduce the amplitude of heart sounds, making it difficult to distinguish the origin of heart sounds.

Distinguishing a sound as systolic or diastolic, and originating from the left or right side of the heart helps a doctor to determine a specific valvular heart disease. Therefore, there is a need to have a method to accurately determine the origin (the left or right side of the heart) of a heart sound and optionally also the precise timing (systolic or diastolic).

### SUMMARY OF THE INVENTION

The invention is defined by the claims.

According to examples in accordance with an aspect of the invention, there is provided a system for identifying a location of the heart responsible for a particular sound such as a heart murmur, comprising:
a physiological sensing system, for:
   identifying heart cycles;
   detecting respiration phases; and
   monitoring heart sounds; and
a controller, wherein the controller is adapted to:
   categorize the identified heart cycles into heart cycles which take place during inspiration and heart cycles which take place during expiration; and
   analyze the heart sounds for the heart cycles which take place during inspiration and the heart sounds for heart cycles which take place during expiration, thereby to determine if the particular heart sound is from the left side or the right side of the heart.

This system performs the analysis of heart sounds, and based on a separate analysis of heart sounds during inspiration and during expiration, it can be determined if a particular heart sound, such as a murmur, relates to the left side or the right side of the heart. This system thus enables a determination of the cause of a heart murmur with reduced requirement for advanced practical examination skills. In particular, differentiating between left and right heart sounds by ear (e.g. using a stethoscope) requires significant skill and training.

For example, left sided murmurs may result in higher intensity sound features during expiration than inspiration, while right sided murmurs may behave oppositely.

The physiological sensing system may be further adapted for identifying the systolic and diastolic time periods of the heart cycles, and the controller is adapted to:
categorize the systolic and diastolic time periods into time periods which take place during inspiration and time periods which take place during expiration; and
analyze the heart sounds for the time periods which take place during inspiration and the heart sounds for time periods which take place during expiration, thereby to determine if the particular heart sound is from the left side or the right side of the heart.

In this way, the system is able to separate sounds into sounds that occur during the systole and sounds that occur during the diastole. The particular sound may thus be analyzed additionally depending on whether it occurs in the diastole or systole. Different causes of particular sounds (e.g. murmurs) may then be more accurately determined.

The system may further comprise a recording system for recording the heart sound, wherein the recording system is configurable to record or play only sounds during the systoles or only sounds during the diastoles.

In this way, a medical practitioner is able to listen separately to the sounds during the diastoles and the sounds during the systoles, so that the analysis is simplified.

The physiological sensing system may comprise, for identifying the heart cycles and optionally systolic and diastolic time periods of those heart cycles:
an ECG system; and/or
a PPG system.

Either or both of these may be used to generate signals which enable identification of the timing of the heart cycles and optionally also different phases of the heart cycles.

The physiological sensing system may comprise, for identifying the respiration phases:
an ECG system; and/or
a respiration sensor.

Either or both of these may be used to generate signals which enable identification of the timing of different phases of the breathing cycle. In some examples, a shared sensing system enables respiration cycle as well as heart cycle analysis.

The physiological sensing system may comprise, for monitoring the heart sounds, an ultrasound transducer microphone system, such as a PMUT or CMUT system. It may for example be based on an array of one or more CMUT cells.

The physiological sensing system may comprise, for monitoring the heart sounds, an electronically steerable microphone array. This may provide additional information or verification of the side of the heart which is generating the particular sound.

A haptic feedback system may be used to indicate whether the particular heart sound is from the left side or the right side of the heart. This allows information to be provided which does not interfere with the heart sounds being listened to by a practitioner.

The controller may be adapted to:
determine a maximum heart sound level for heart cycles which take place during inspiration;
determine a maximum heart sound level for heart cycles which take place during expiration; and
determine if the particular heart sound is from the left side or the right side of the heart based on analysis of the maximum heart sound levels.

In this way, an increased maximum heart sound is used as an identifier of the presence of the particular sound. It is then determined if it arises or is more dominant during inspiration or during expiration. This provides an indicator of whether the left or right side of the heart is responsible.

The controller may be adapted to:
determine a first average of the maximum heart sound level for heart cycles which take place during inspiration;
determine a second average of the maximum heart sound level for heart cycles which take place during expiration; and
determine if the particular heart sound is from the left side or the right side of the heart based on a comparison of the first and second average.

This provides one particular processing approach based on an average of the maximum heart sound level in each heart cycle being analyzed.

The invention also provides a method for identifying a location of the heart responsible for a particular heart sound such as a heart murmur, comprising:
identifying heart cycles;
detecting respiration phases;
monitoring heart sounds;
categorizing the identified heart cycles into heart cycles which take place during inspiration and heart cycles which take place during expiration; and
analyzing the heart sounds for the heart cycles which take place during inspiration and the heart sounds for heart cycles which take place during expiration, thereby to determine if the particular heart sound is from the left side or the right side of the heart.

This method is implemented by the device defined above. It is based on separate analysis of heart sounds during inspiration and during expiration.

The method may comprise:
identifying the systolic and diastolic time periods of the heart cycles;
categorizing the systolic and diastolic time periods into time periods which take place during inspiration and time periods which take place during expiration; and
analyzing the heart sounds for the time periods which take place during inspiration and the heart sounds for time periods which take place during expiration, thereby to determine if the particular heart sound is from the left side or the right side of the heart.

The method may comprise:
determining a maximum heart sound level for heart cycles which take place during inspiration;
determining a maximum heart sound level for heart cycles which take place during expiration; and
determining if the particular heart sound is from the left side or the right side of the heart based on analysis of the maximum heart sound levels.

For example, a first average of the maximum heart sound level may be obtained for heart cycles which take place during inspiration, a second average of the maximum heart sound level may be obtained for heart cycles which take place during expiration, and the particular heart sound is determined to be from the left side or the right side of the heart based on a comparison of the first and second average.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for identifying a location of the heart responsible for a particular sound such as a heart murmur;
Figure 2 shows an Electrocardiography (ECG) waveform;
Figure 3 shows the relationship between an ECG signal and a photoplethysmography (PPG) signal;
Figure 4 shows heart sounds which arise during the heart cycle;
Figure 5 shows a respiration signal in addition to ECG and PPG signals;
Figure 6 shows examples of normal and abnormal heart sounds;
Figure 7 shows an example of processing method for identifying a location of the heart responsible for a particular sound; and
Figure 8 which shows how a directional microphone array may be used.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for identifying a location of the heart responsible for a particular sound such as a heart murmur. Heart sounds are analyzed separately for heart cycles which take place during inspiration and during expiration. Based on the separate analysis, it is possible to determine if a particular heart sound is from the left side or the right side of the heart.

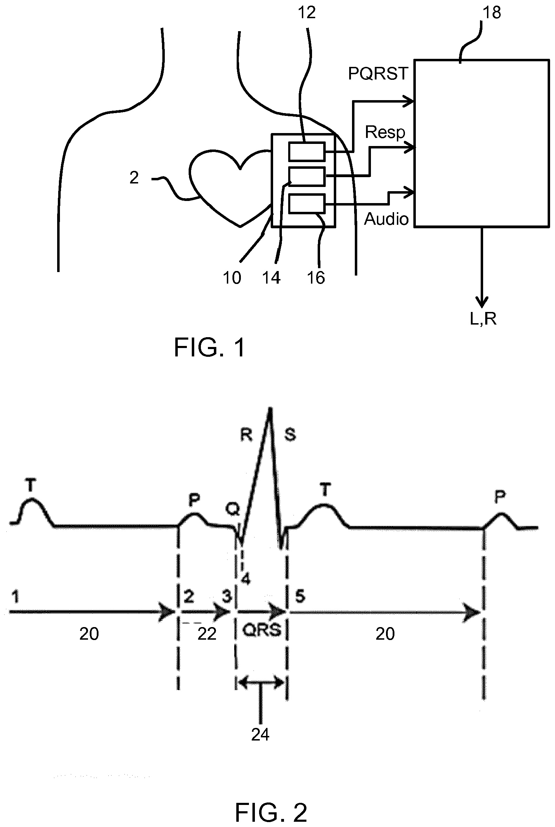

Figure 1 shows a system for identifying a location of the heart 2 responsible for a particular sound such as a heart murmur.

The system comprises a physiological sensing system 10.

This comprises a first sensing unit 12 for identifying heart cycles (e.g. the PQRST points of an ECG), a second sensing unit 14 for detecting respiration phases ("Resp") and a third unit 16 for monitoring heart sounds ("Audio") i.e. for auscultation.

The sensing system may have one or more sensing units, since some sensing modalities may be able to perform two or more of the different sensing functions. Thus, the sensing system 10 is shown with separate units 12, 14, 16 simply for ease of explanation. In practice, all that is required is that the cycles of the heart as well as the breathing cycles can be identified, and that there is an audio sensor detecting heart sounds. The invention is for example for identifying the location (and optionally also timing) of particular sounds, such as heart murmur sounds.

Examples of possible physiological sensing modalities are discussed below.

A controller 18 categorizes the identified heart cycles into heart cycles which take place during inspiration and heart cycles which take place during expiration, by using the respiration phase information.

The heart sounds are then analyzed for the heart cycles which take place during inspiration and the heart sounds for heart cycles which take place during expiration, thereby to determine if the particular heart sound is from the left side or the right side of the heart. This information is provided as the output shown as L,R. As also discussed below, this output may take various forms.

By way of example, multiple physiological signals are measured, such as heart sounds, an ECG trace, a PPG signal and a respiratory sensor signal. The signals are analyzed together to enable automatic differentiation between left and right heart sounds. After the analysis, the system can give feedback to a medical practitioner. The heart sound signals are for example obtained with a (localized) acoustic recording device. Characteristic points in an ECG trace and in a PPG signal may be used for determining the systolic and diastolic periods. Characteristic points (the peaks and the troughs) may be identified in the respiration signal for determining left or right sidedness of murmurs and characterizing the split of heart sounds. The output may be in the form of haptic feedback for left and right heart sounds. Visual output is also possible, such as a color-coded output. The system in this way alleviates problems of inexperienced doctors.

A simple acoustic device such as an (electronic) stethoscope can be used for the audio signal collection. However, an electronically steerable microphone array may be used to provide sound localization. Different parts of the array will then be able to pick up sounds from different sides and regions of the heart, e.g. the right valve. A microphone array may also give the ability to localize a signal using a phase difference (delay and sum) approach. For heart valves, a low resolution implementation will suffice (due to the lack of many high frequency components in the heart valve sounds). A microphone array may also give an ability to reduce noise.

A microphone array may for example be arranged around a central ECG electrode or ECG electrode pair.

Various possible physiological sensing approaches will now be discussed, which may be used to implement the system of Figure 1, or may be used to supplement the information from the system of Figure 1.

For detecting heart sounds, i.e. auscultation, an (electronic) stethoscope is generally used. Cardiac auscultation constitutes an important part of clinical medicine. Recently, techniques associated with advances in electronic stethoscopes have been developed to process auscultatory heart sound signals as well as analyze and clarify the resulting sounds, to make diagnosis based on quantifiable medical assessments.

Capacitive micromachined ultrasonic transducers (CMUT) or piezoelectric micromachined ultrasonic transducers (PMUT) are relatively new concepts in the field of ultrasonic transducers, for detecting and measuring incident sounds. Most commercial ultrasound transducers today are based on piezoelectric sensors. For example, CMUTs are constructed on silicon using micromachining techniques. A cavity is formed in a silicon substrate, and a thin layer suspended on the top of the cavity serves as a membrane on which a metallized layer acts an electrode, together with the silicon substrate which serves as a bottom electrode. As CMUTs are micromachined devices, it is easier to construct 2D and 3D arrays of transducers using this technology. This means large numbers of CMUTs can be included in a transducer array providing larger bandwidth compared to other transducer technologies. A high frequency operation using CMUTs is easy to achieve due to the small dimensions. The frequency of operation depends on the cell size and on the stiffness of the material used as a membrane.

Figure 2 shows an Electrocardiography (ECG) waveform. Region 20 is the early diastole, region 22 is the late diastole and region 24 is the systole.

An ECG records the electrical activity of the heart over a period of time using electrodes placed on the skin. These electrodes detect the tiny electrical changes on the skin that arise from the heart muscle's electrophysiological pattern of depolarizing and repolarizing during each heartbeat. It is very commonly performed to detect any cardiac problems.

In Figure 2, the various zones of interest are identified as follows:
P is the atrial systole contraction pulse;
Q is a downward deflection immediately preceding the ventricular contraction;
R is the peak of the ventricular contraction;
S is the downward deflection immediately after the ventricular contraction;
T is the recovery of the ventricles.

A photoplethysmogram (PPG) is an optically obtained plethysmogram, a volumetric measurement of an organ. A PPG is often obtained by using a pulse oximeter which illuminates the skin and measures changes in light absorption. A conventional pulse oximeter monitors the perfusion of blood to the dermis and subcutaneous tissue of the skin. With each cardiac cycle, the heart pumps blood to the periphery. Even though this pressure pulse is somewhat damped by the time it reaches the skin, it is enough to distend the arteries and arterioles in the subcutaneous tissue. If the pulse oximeter is attached without compressing the skin, a pressure pulse can also be seen from the venous plexus, as a small secondary peak.

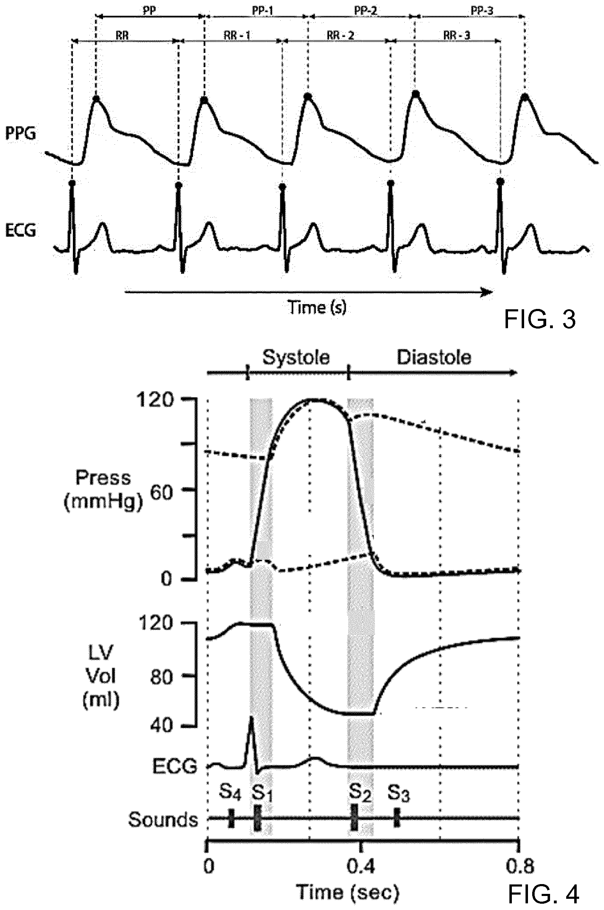

Figure 3 shows the relationship between an ECG signal and a PPG signal.

Either of these may be used to identify the heart cycles. The systoles and diastoles can for example be automatically determined once the characteristic points PQRST of the ECG are detected. The PPG signal may be used as an additional validation step, to rectify the systolic and diastolic intervals. The systoles and diastoles can be for example determined from the PPG signal by detecting the systolic peak and the diastolic peak.

For detecting the respiration phases, a dedicated respiration sensor may be used, but respiration sensing can also be derived from both the ECG and PPG signals using amplitude modulation (AM), frequency modulation (FM), and baseline wander (BW).

Figure 4 shows heart sounds which arise during the heart cycle. It shows an a ventricular pressure plot (Press) at the top, a left ventricular volume plot (LV Vol) second, an ECG plot (ECG) third, and a sound plot (Sounds) at the bottom.

Sound S1 is the "lupp" sound at the start of the systole, and sound S2 is the "dubb" sound at the start of the diastole. Sounds S3 and S4 are less common heart sounds, which may be heard in both normal and abnormal situations.

Once the systoles and diastoles are detected, differentiation between the heart sounds S1 and S2 becomes trivial for patients with normal heart beat. However, in presence of tachycardia or in presence of the S3 and S4 sounds or other additional heart sounds, the detection of the source of the sound becomes critical. In such cases, the invention facilitates narrowing down the source of a particular heart sound.

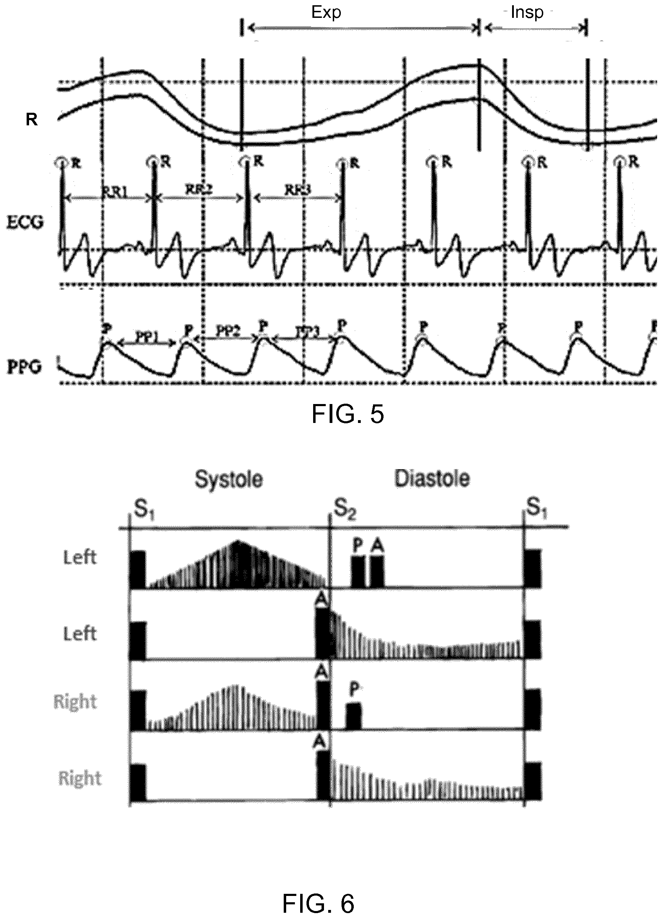

Figure 5 shows a respiration signal (R) in addition to the ECG and PPG signals. A respiratory sensor is used to detect the respiratory cycle using a peak and trough detection algorithm based on the slopes of the signal.

A respiration sensor may for example be a respiration belt sensor (for example one on the chest and one on the stomach), a breath sensor or a sensor based on the capture of camera images of the chest/lung. Ballistographic approaches are also possible, or even motion detectors in equipment contacted by the subject, such as a bed, couch or chair.

Figure 5 for example shows two respiration signals, one from a stomach belt and one from a chest belt.

Alternatively, the respiration signal may be obtained using known processing of ECG or PPG signals.

Figure 6 shows examples of abnormal heart sounds. A normal heart sound would comprise the sounds S1 and S2.

The first plot shows the sound for aortic stenosis. There is additional sound in the systole from the left side of the heart.

The second plot shows the sound for aortic regurgitation. There is additional sound in the diastole from the left side of the heart.

The third plot shows the sound for pulmonic stenosis. There is additional sound in the systole from the right side of the heart.

The forth plot shows the sound for pulmonic regurgitation. There is additional sound in the diastole from the right side of the heart.

The additional sounds from the left side will occur more during expiration and the additional sounds from the right side will occur more during inspiration. This is because venous return from systemic veins (superior and inferior vena cava) increase during inspiration due to increased negative pressure in the thoracic cavity resulting in increased regurgitation/turbulence through tricuspid and pulmonary valves (i.e. valves belonging to right side of the heart). This increases the loudness of murmurs resulting from pathologies of these valves. On the other hand, pulmonary venous return increases during expiration causing a similar increase in loudness of murmurs resulting from mitral and aortic valve pathologies (i.e. from left side of the heart).

By detecting the timing of the systole and diastole, as well as the left side or right side location (using the respiration information), more accurate diagnosis of the possible heart conditions is made possible.

Thus, by accurately detecting the systoles and diastoles as well as the respiratory cycle, a recorded murmur can be characterized based on whether it appears during the systole or the diastole as well as the intensity profile during the cardiac cycle (e.g. early systolic, pan-systolic, crescendo-decrescendo, early diastolic etc.). The sound source may be located using either a single microphone or a microphone array with its own sound localization capability. Knowledge of the typical radiation patterns of murmurs may also be used in a characterization process.

When the microphone array already provides some (low resolution) localization information, the respiratory cycle information is utilized to further improve the confidence of this localization and characterization, based on left sided murmurs being of higher intensity during expiration than inspiration while right sided murmurs behave oppositely.

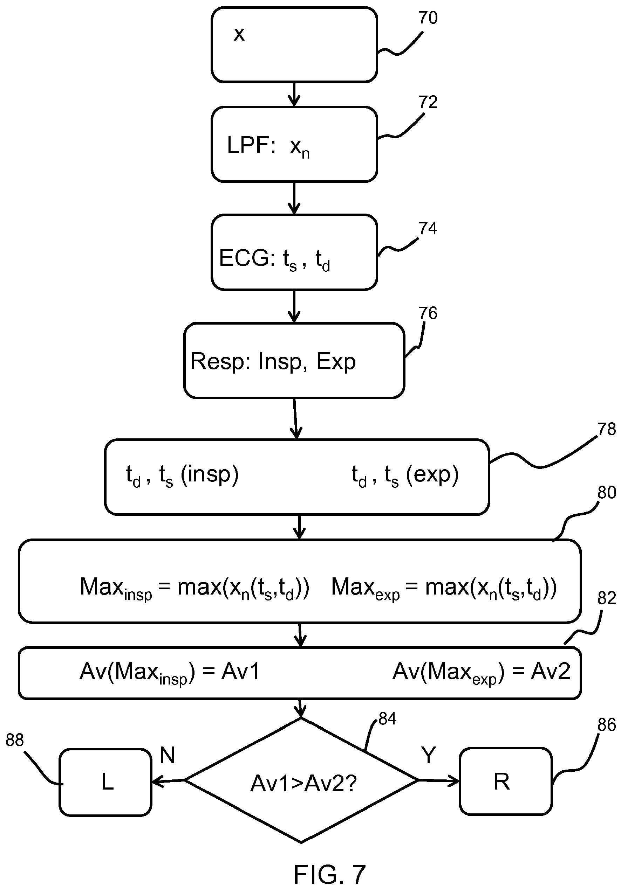

Figure 7 shows an example of processing method.

In step 70, a sound signal x is obtained, for example from a single microphone or a microphone array. The microphone is for example based on CMUT technology.

In step 72, the sound signal is filtered, for example with a low pass filter, for example with a cutoff frequency of 600Hz. The filtered sound signal may then be normalized. The result is a filtered sound signal xₙ.

In step 74, an ECG signal is obtained. From this, the systole tₛ and diastole t_{d} are identified.

In step 76 a respiration signal is obtained. This may be derived from the ECG signal or obtained by a dedicated sensor. The inspiration "Insp" and expiration "Exp" phases are identified.

These steps basically provide the physiological signals needed for the subsequent processing. They may be collected simultaneously, or they may be collected and stored in any order. The sound signals may subsequently be synchronized with the respiration phases if they are obtained at different times.

In step 78 each pair of systoles and diastoles (i.e. each individual heart cycle) is classified as occurring during the inspiration phase or the expiration phase. This is for example achieved by comparing the respiration signal with thresholds to determine when in the different respiration phases.

For the cycles during the inspiration phase, a maximum sound signal is determined Maxᵢₙₛₚ=max(xₙ(tₛ,t_{d})). This may be carried out for all cycles within an analysis period or for a subset of cycles. Each cycle is made up of a systole (time period tₛ) and a diastole (time period t_{d}).

The process may use a subset of the cycles, for example by ignoring heart cycles close to the transitions between inspiration and expiration.

Similarly, for the cycles during the expiration phase, a maximum sound signal is determined Maxₑₓₚ=max(xₙ(tₛ,t_{d})). Again, this may be carried out for all cycles within an analysis period or for a subset of cycles.

In step 82, a first average Av1 is taken of the maximum inspiration amplitudes, and a second average Av2 is taken of the maximum expiration amplitudes.

In step 84, the two averages are compared.

The source of the sound being located is determined in step 86 as from the right side of the heart if the first (inspiration) average Av1 is greater than the second (expiration) average Av2. The source of the sound being located is determined in step 88 as from the left side of the heart if the first (inspiration) average Av1 is less than the second (expiration) average Av2.

The detection of the systoles or diastoles may be used to selectively record heart sounds from either the systole or the diastole. This information could be very important for medical practitioners as different cardiac pathologies have murmurs in different phases of the cardiac cycle. However, given a very short duration of the heart cycle (frequently 1 second or less) medical practitioners usually find it difficult to determine the correct phase of the murmur. The selective recording could also help a medical practitioner to judge characterization of murmur in relation to a particular phase (e.g. early systolic, end systolic or continuous) without any disturbance of heart sounds from the other phase of cardiac cycle.

As mentioned above, haptic feedback may be used to identify the systoles and diastoles and/or to identify right and left sides of the heart. Haptic feedback of different duration or frequency may be used to indicate that a left or right sided murmur has been identified. The haptic feedback can for example be provided to the user as a form of vibration in the measurement device itself (i.e. the physiological sensor system 10) while performing the measurements. This does not distort the heart sound in any manner, whilst still alerting the user to the systole or diastole.

As an alternative, once the left and right sided murmurs are accurately detected as described above, a color-coded visual feedback or else a text message or annotation may be used. This feedback can be both real-time and offline depending on the streaming recording methodology.

As mentioned above, microphone array may be used, to implement a directional microphone. This may provide initial direction information.

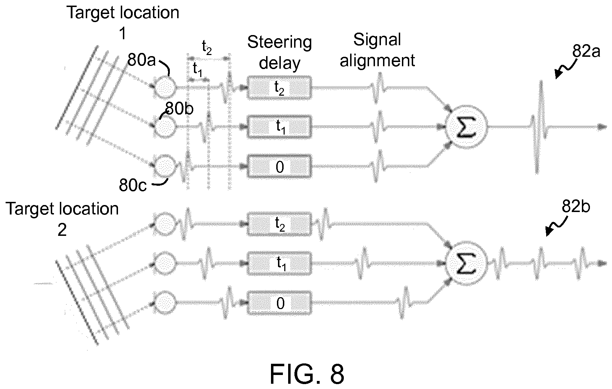

The steering and focusing of a directional microphone array may be achieved in a similar way to that used for ultrasound beamforming; in particular, by use of a delay- and-sum (DAS) beamforming applied to the received acoustic signals of the array of sound sensors. This is illustrated in Figure 8, which shows how signals received at an example ordered row of microphones 80a, 80b, 80c, may be compiled together with different patterns of signal steering delays (t₀, t₁, t₂) imposed in order to generate output composite signals 82a, 82b corresponding to different spatial directions. This implements electronic steering of the microphone array.

In the upper image, a first set of time delays is applied between the compiled signals, leading to an effective directional focus toward target source location 1. In the lower image, a second set of time delays is applied between the compiled signals, leading to an effective focus in target source location 2.

The microphones may be ultrasound CMUT devices. The desired range of the heart sounds to be analyzed, such as in the Hz to kHz range, may be implemented by increasing the mass of a CMUT membrane compared to a design for MHz ultrasound imaging frequencies. This additional mass lowers the resonant frequency. A shift in frequency sensitivity compared to a MHz design may also be achieved by changing a membrane area, membrane thickness, or voltage bias.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

Where the term "adapted to" used in the claims and description, it is noted the term "adapted to" is intended to be equivalent to the term "configured to". Any reference signs in the claims should not be construed as limiting the scope.

## Claims

1. A system for identifying a location of the heart responsible for a particular sound such as a heart murmur, comprising:
a physiological sensing system (10), for:
identifying heart cycles;
detecting respiration phases; and
monitoring heart sounds; and
a controller (18), wherein the controller is adapted to:
categorize the identified heart cycles into heart cycles which take place during inspiration and heart cycles which take place during expiration; and
analyze the heart sounds for the heart cycles which take place during inspiration and the heart sounds for heart cycles which take place during expiration, thereby to determine if the particular heart sound is from the left side or the right side of the heart.

2. A system as claimed in claim 1, wherein the physiological sensing system (10) is further for identifying the systolic and diastolic time periods of the heart cycles, and the controller (18) is adapted to:
categorize the systolic and diastolic time periods into time periods which take place during inspiration and time periods which take place during expiration; and
analyze the heart sounds for the time periods which take place during inspiration and the heart sounds for time periods which take place during expiration, thereby to determine if the particular heart sound is from the left side or the right side of the heart.

3. A system as claimed in claim 2, further comprising a recording system for recording the heart sounds.

4. A system as claimed in claim 3, wherein the recording system is configurable to record or play only sounds during the systoles or only sounds during the diastoles.

5. A system as claimed in any one of claims 1 to 4, wherein the physiological sensing system comprises, for identifying the heart cycles and optionally systolic and diastolic time periods of those heart cycles:
an ECG system (12); and/or
a PPG system.

6. A system as claimed in any one of claims 1 to 5, wherein the physiological sensing system comprises, for identifying the respiration phases:
an ECG system; and/or
a respiration sensor (14).

7. A system as claimed in any one of claims 1 to 6, wherein the physiological sensing system comprises, for monitoring the heart sounds, a CMUT or PMUT microphone system (16), for example an electronically steerable microphone array.

8. A system as claimed in any one of claims 1 to 7, further comprising a haptic feedback system for indicating whether the particular heart sound is from the left side or the right side of the heart.

9. A system as claimed in any one of claims 1 to 8, wherein the controller (18) is adapted to:
determine a maximum heart sound level for heart cycles which take place during inspiration;
determine a maximum heart sound level for heart cycles which take place during expiration; and
determine if the particular heart sound is from the left side or the right side of the heart based on analysis of the maximum heart sound levels.

10. A system as claimed in claim 9, wherein the controller is adapted to:
determine a first average of the maximum heart sound level for heart cycles which take place during inspiration;
determine a second average of the maximum heart sound level for heart cycles which take place during expiration; and
determine if the particular heart sound is from the left side or the right side of the heart based on a comparison of the first and second average.

11. A method for identifying a location of the heart responsible for a particular heart sound such as a heart murmur, comprising:
(74) identifying heart cycles;
(76) detecting respiration phases;
(70) monitoring heart sounds;
(78) categorizing the identified heart cycles into heart cycles which take place during inspiration and heart cycles which take place during expiration; and
(80,82,84,86,88) analyzing the heart sounds for the heart cycles which take place during inspiration and the heart sounds for heart cycles which take place during expiration, thereby to determine if the particular heart sound is from the left side or the right side of the heart.

12. A method as claimed in claim 11, comprising:
(74) identifying the systolic and diastolic time periods of the heart cycles;
(78) categorizing the systolic and diastolic time periods into time periods which take place during inspiration and time periods which take place during expiration; and
(80,82,84,86,88) analyzing the heart sounds for the time periods which take place during inspiration and the heart sounds for time periods which take place during expiration, thereby to determine if the particular heart sound is from the left side or the right side of the heart.

13. A method as claimed in claim 11 or 12, comprising:
(80) determining a maximum heart sound level for heart cycles which take place during inspiration;
(80) determining a maximum heart sound level for heart cycles which take place during expiration; and
(84) determining if the particular heart sound is from the left side or the right side of the heart based on analysis of the maximum heart sound levels.

14. A method as claimed in claim 13, comprising:
(82) determining a first average of the maximum heart sound level for heart cycles which take place during inspiration;
(82) determining a second average of the maximum heart sound level for heart cycles which take place during expiration; and
(84) determining if the particular heart sound is from the left side or the right side of the heart based on a comparison of the first and second average.

15. A computer program comprises computer program code means which is adapted, when said program is run on a computer, to implement the method of any one of claims 11 to 14.
